(19) Europäisches Patentamt
European Patent Office
Office européen des brevets

(11) Veröffentlichungsnummer: **0 071 920**
**B1**

(12) # EUROPÄISCHE PATENTSCHRIFT

(45) Veröffentlichungstag der Patentschrift: **11.07.84**

(51) Int. Cl.³: **C 07 C 69/708,** C 07 C 67/37, C 07 C 69/675

(21) Anmeldenummer: **82106931.7**

(22) Anmeldetag: **31.07.82**

(54) **Verfahren zur Carbonylierung von Formaldehyd-acetalen.**

(30) Priorität: **07.08.81 DE 3131355**

(43) Veröffentlichungstag der Anmeldung: **16.02.83 Patentblatt 83/7**

(45) Bekanntmachung des Hinweises auf die Patenterteilung: **11.07.84 Patentblatt 84/28**

(84) Benannte Vertragsstaaten: **BE DE FR GB NL**

(56) Entgegenhaltungen:
**DE - A - 1 192 178**
**US - A - 2 273 269**

(73) Patentinhaber: **HOECHST AKTIENGESELLSCHAFT, Postfach 80 03 20, D-6230 Frankfurt am Main 80 (DE)**

(72) Erfinder: **Schmidt, Hans-Joachim, Dr., Am Burgenblick 6, D-6240 Königstein/Taunus (DE)**
Erfinder: **Arpe, Hans-Jürgen, Prof.Dr., de-Ridder-Weg 10, D-6230 Frankfurt am Main 80 (DE)**

**EP 0 071 920 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Umsetzung von Formaldehyd-acetalen der allgemeinen Formel

$RO-CH_2-OR$

worin die beiden Reste R gleich oder verschieden sein können und Alkyl-, Alkoxyalkyl-, Cycloalkyl- oder Phenylalkylgruppen mit bis zu 10 Kohlenstoffatomen bedeuten, mit Kohlenmonoxid in der Flüssigphase bei erhöhten Temperaturen und Drücken.

Bei der erfindungsgemässen Umsetzung der Formaldehyd-acetale mit Kohlenmonoxid entstehen hauptsächlich Ether-Ester der Hydroxyessigsäure (Glykolsäure) nach der allgemeinen Gleichung (1), worin R die oben genannte Bedeutung hat:

$$RO-CH_2-OR+CO \longrightarrow RO-CH_2-COOR \qquad (1)$$

Die Verbindungen der Formel $RO-CH_2-COOR$ werden im folgenden allgemein als «Ether-Ester der Hydroxyessigsäure» bezeichnet. Neben diesen Verbindungen können unter den Reaktionsbedingungen in geringen Mengen durch Nebenreaktionen, z.B. Hydrolyse, auch Ester der Hydroxyessigsäure (Glykolsäure) der Formel $HO-CH_2-COOR$, sowie solche Verbindungen auftreten, die formal durch Etherabspaltung aus zwei Molekülen von Ether-Estern der Hydroxyessigsäure nach Gleichung (2) entstanden sein können:

$$RO-CH_2COOR+RO-CH_2COOR \longrightarrow RO-CH_2COO-CH_2-COOR+R_2O \qquad (2)$$

Es ist bereits bekannt, Formaldehyd-acetale mit Kohlenmonoxid in Gegenwart von Lewissäure-Katalysatoren wie Bortrifluorid (US-PS 2 273 269) oder Fluorwasserstoff (US-PS 3 948 977) unter Bildung von Alkoxyessigsäurealkylestern umzusetzen. Diese bekannten Verfahren erfordern jedoch einen unwirtschaftlich grossen Überschuss an Katalysator, beispielsweise 27–75 Mol-% $BF_3$ oder weit über 100 Mol-% HF pro Mol eingesetztes Acetal. Die Rückgewinnung dieser in Substanz leicht flüchtigen und im Reaktionsmedium homogen verteilten Katalysatoren ist aber schwierig und macht aufwendige Massnahmen erforderlich.

Es ist aus der deutschen Offenlegungsschrift 2 617 085 weiter bekannt, Aldehyde mit Kohlenmonoxid und Wasser oder Alkoholen oder Carbonsäuren in Gegenwart saurer Ionenaustauscher zu Hydroxycarbonsäuren oder deren Estern oder Acylderivaten umzusetzen (Gleichungen 3–5):

$$R-CHO+CO+H_2O \longrightarrow HO-\overset{\displaystyle R}{\overset{|}{C}}H-COOH \qquad (3)$$

$$R-CHO+CO+R^1OH \longrightarrow HO-\overset{\displaystyle R}{\overset{|}{C}}H-COOR^1 \qquad (4)$$

$$R-CHO+CO+R^2COOH \longrightarrow R^2COO-\overset{\displaystyle R}{\overset{|}{C}}H-COOH \qquad (5)$$

Nach diesen Verfahren werden aber nur Monoderivate, d.h. entweder an der Hydroxyl- oder an der Carboxylgruppe substituierte Hydroxycarbonsäuren erhalten.

Es wurde nun gefunden, dass sich Formaldehyd-acetale in einfacher Weise zu Ether-Estern der Hydroxyessigsäure carbonylieren lassen, wenn man die Umsetzung in Gegenwart saurer organischer Ionenaustauscher durchführt. Die Verwendung von organischen Ionenaustauschern als Carbonylierungskatalysator hat den grossen Vorteil, dass man den Katalysator leicht abtrennen und wiederverwenden oder die Reaktion an einem stationär angeordneten Katalysator kontinuierlich durchführen kann.

Gegenstand der Erfindung ist daher ein Verfahren zur Umsetzung von Formaldehyd-acetalen der allgemeinen Formel

$RO-CH_2-OR$

worin die beiden Reste R gleich oder verschieden sein können und Alkyl-, Alkoxyalkyl-, Cycloalkyl- oder Phenylalkylgruppen mit bis zu 10 Kohlenstoffatomen bedeuten, mit Kohlenmonoxid in der Flüssigphase bei erhöhten Temperaturen und Drücken, dadurch gekennzeichnet, dass man als Katalysator saure organische Ionenaustauscher mit einer Austauscherkapazität von mehr als 0.5 mval pro g verwendet.

Der Befund, dass sich die Acetale des Formaldehyds in Gegenwart von sauren organischen Ionenaustauschern carbonylieren lassen, war überraschend, da beispielsweise aus der deutschen Auslegeschrift 1 668 482 bekannt ist, dass bei Acetalen in Gegenwart von sauren Ionenaustauschern beide Acetalbindungen stark labilisiert werden und so z.B. mit nucleophilen Reaktionspartnern unter Rückbildung des Einsatzaldehyds die Alkylgruppen leicht abgespalten werden. Analog war im Fall der erfindungsgemässen Umsetzung mit der Bildung von Formaldehyd, Dialkylethern und anderen Produkten zu rechnen, es war aber nicht zu erwarten, dass Kohlenmonoxid in so glatter Weise in das Acetalmolekül eingebaut wird.

Formaldehyd-acetale, die nach dem erfindungsgemässen Verfahren mit Kohlenmonoxid umgesetzt werden können, entsprechen der allgemeinen Formel $RO-CH_2OR$, worin die beiden Reste R unabhängig voneinander Alkyl-, Alkoxyalkyl-, Cycloalkyl- oder Phenylalkylgruppen mit bis zu 10 Kohlenstoffatomen bedeuten. Besonders geeignet sind die Dimethyl-, Diethyl, Di-n-propyl-, Di-isobutyl-, Di-(2-ethoxy-ethyl)-, Di-cyclohexyl-, Di-benzyl- und Di-phenylethyl-acetale des Formaldehyds, ferner gemischte Formaldehydacetale wie das Methylethyl- oder das Ethylpropylformal.

Die erfindungsgemäss als Katalysatoren verwendeten sauren organischen Ionenaustauscher sind handelsübliche, auf der Basis kernsulfonierter Copolymerisate aus Styrol oder Divinylbenzol oder auf der Basis perfluorsulfonierter Polymethylene aufgebaute Kationenaustauscher mit freien Sulfonsäuregruppen und Austauscherkapazitäten von mehr als 0.5 mval pro Gramm. Bevorzugt sind stark saure organische Ionenaustauscher mit Austauscherkapazitäten von mehr als 1 mval pro Gramm.

Der Wassergehalt des Austauschers kann in weiten Grenzen schwanken. Um Nebenproduktbildung durch Hydrolyse des eingesetzten Acetals bzw. des gebildeten Ether-Esters der Hydroxyessigsäure zu vermeiden, ist es jedoch vorteilhaft, Ionenaustauscher mit einem Wassergehalt von weniger als 5 Gew.-% zu verwenden. Ionenaustauscher mit höherem Wassergehalt lassen sich nach bekannten Methoden entwässern, beispielsweise durch Trocknen in der Wärme oder im Vakuum, durch Waschen mit einem hydrophilen Lösungsmittel wie Isopropanol, Essigsäure oder Aceton, durch Umsetzung mit Acetanhydrid, sowie durch Azeotropdestillation, z.B. mit Benzol.

Die Formaldehyd-acetale lassen sich unverdünnt oder in Mischung mit einem oder mehreren Lösungs- oder Verdünnungsmitteln einsetzen. Geeignet sind hierfür beispielsweise aliphatische oder aromatische Kohlenwasserstoffe, Ether, Carbonsäureester sowie ggf. auch der bei der Carbonylierung entstehende Ether-Ester der Hydroxyessigsäure selbst.

Das Gewichtsverhältnis von Ionenaustauschern zu den Formaldehyd-acetalen kann in weiten Grenzen variiert werden. Da die verwendeten Ionenaustauscher wegen ihrer Korngrösse und Unlöslichkeit leicht vom Reaktionsgemisch abgetrennt werden können, andererseits die Umsetzgeschwindigkeit des Acetals mit steigender Austauscherkonzentration zunimmt, ist es zweckmässig, nicht zu geringe Konzentrationen zu wählen. Bevorzugt sind Austauscherkonzentrationen von mehr als 1 Gew.-%, bezogen auf die eingesetzte Reaktionslösung, d.h. bezogen auf die Summe aus dem eingesetzten Formal und ggf. dem Lösungs- oder Verdünnungsmittel.

Die Aufarbeitung des Reaktionsgemisches erfolgt im allgemeinen destillativ. Gegebenenfalls können vor oder während der Destillation (durch Umsetzung des Reaktionsgemischs mit einem Alkohol in Gegenwart des Ionenaustauschers) die nach Gleichung (2) gebildeten Verbindungen quantitativ in äquimolare Mengen Ether-Ester der Hydroxyessigsäure und Glykolsäureester gespalten werden. Zweckmässig verwendet man dabei einen Alkohol, der dem eingesetzten Formaldehyd-acetal entspricht, beispielsweise Methylalkohol im Falle von Formaldehyd-dimethylacetal. Das Reaktionsgemisch enthält nach dieser Behandlung als Carbonylierungsprodukte nur noch den genannten Ether-Ester und Glykolsäureester, deren Trennung einfach ist.

Zur Durchführung des erfindungsgemässen Verfahrens wird das Acetal in geeigneter Weise mit Kohlenmonoxid und dem Ionenaustauscher in Berührung gebracht, beispielsweise in Druckgefässen mit Rühr-, Hub- oder Schüttelvorrichtung oder bei kontinuierlichem Betrieb vorteilhaft in Strömungsrohren mit stationär angeordnetem Katalysator.

Bei Arbeiten in Druckgefässen wird, ggf. nach Spülen mit Stickstoff, das Kohlenmonoxid in üblicher Weise dem Reaktionsgemisch zugeführt, zum Beispiel aus Druckflaschen oder mittels Pumpe. Mit fortschreitender Umsetzung und bei entsprechendem Druckabfall kann das verbrauchte Kohlenmonoxid in gleicher Weise ergänzt werden. Bei kontinuierlicher Arbeitsweise werden das Acetal und das Kohlenmonoxid der Reaktionszone dauernd zugeführt und eine entsprechende Menge Reaktionsgemisch und Restgas entnommen.

Eine bevorzugte kontinuierliche Ausführungsform des erfindungsgemässen Verfahrens besteht darin, den Ionenaustauscher als Fest- oder Fliessbett in einem Strömungsrohr anzuordnen und das Acetal, ggf. verdünnt mit einem Lösungsmittel, sowie das Kohlenmonoxid durch das Austauscherbett zu leiten, wobei das Zudosieren der Einsatzprodukte auch an verschiedenen Stellen der Austauscherschicht erfolgen kann.

Die Umsetzungstemperatur liegt im allgemeinen zwischen 25 und 200°C, vorzugsweise zwischen 50 und 180°C, wobei die obere Temperaturgrenze jedoch durch die Art des verwendeten Austauschers bestimmt wird. Bei stark sauren Ionenaustauschern auf der Basis von Styrol-Divinylbenzol-Copolymerisaten liegt die obere Temperaturgrenze im allgemeinen bei 120–140°C, bei perfluorsulfonierten Polymethylenen etwa bei 170–180°C.

Für die Umsetzung werden Drücke zwischen 10 und 300 bar, bevorzugt zwischen 50 und 250 bar angewandt. Druck und Temperatur werden so gewählt, dass das eingesetzte Acetal sowie ggf. das Lösungs- oder Verdünnungsmittel unter den Reaktionsbedingungen in flüssiger Phase vorliegen.

Nach der Umsetzung trennt man in geeigneter Weise, beispielsweise durch Filtration oder Dekantieren, vom unlöslichen Ionenaustauscher ab und isoliert die Reaktionsprodukte durch fraktionierte Destillation.

Die Ether-Ester der Hydroxyessigsäure und Glykolsäureester finden vielseitige Verwendung als Zwischenprodukte und als Lösungsmittel.

Die Erfindung wird durch die nachstehenden Beispiele erläutert.

Beispiel 1

Ein 200 ml Schüttelautoklav wird mit 50 g Formaldehyd-dimethylacetal (Methylal) und 10 g eines handelsüblichen makroretikulären Ionenaustauscherharzes in $H^+$-Form (Wassergehalt 1,2 Gew.-%, Austauscherkapazität 4.6 mval/g) gefüllt. Nach dem Spülen mit Stickstoff wird ein Kohlenmonoxiddruck von 140 bar eingestellt und der Autoklav unter Schütteln 4 Stunden auf 130°C erhitzt. Bei Druckabfall wird durch Zugabe von frischem Kohlenmonoxid der Ausgangsdruck wieder eingestellt. Nach dem Erkalten ergibt die gaschromatographische Untersuchung des Reaktionsgemisches einen Methylal-Umsatz von 95 Mol-% bei einer Selektivität zu Methoxyessigsäuremethylester von 53%, zu Glykolsäuremethylester von 18,5% und zu Methoxyacetyl-glykolsäuremethylester von 22,2%.

Das Reaktionsgemisch wird filtriert und der Ionenaustauscher zweimal mit je 10 g Methylal gewaschen. Bei der Destillation der vereinigten Filtrate über eine 80-cm-Füllkörperkolonne werden 50 g Methoxyessigsäuremethylester vom Kp 130–131°/1 bar, 14 g Glykolsäuremethylester vom Kp 63–64°C/28 mbar und 16 g Methoxyacetylglykolsäuremethylester vom Kp 64–65°C/0,13 mbar erhalten.

Der abfiltrierte Ionenaustauscher lässt sich noch mehrmals zur Carbonylierung des Methylals verwenden. Nach insgesamt fünfmaliger Verwendung liegt der Methylal-Umsatz noch über 90% bei praktisch unveränderten Selektivitäten.

Beispiel 2

Ein 2-Liter-Rührautoklav wird mit 500 g Methylal, 500 g Methylacetat und 50 g des in Beispiel 1 genannten Ionenaustauschers gefüllt. Nach dem Spülen mit Stickstoff gibt man Kohlenmonoxid bis zu einem Druck von 140 bar zu und erhitzt bei einer Rührgeschwindigkeit von 1000 Umdrehungen pro Minute 3 Stunden auf 130°C. Der Druckabfall bei der Reaktion wird durch Zugabe von frischem Kohlenmonoxid kompensiert. Nach dem Erkalten und Entspannen enthält das Reaktionsgemisch 39,4 Gew.-% Methoxyessigsäuremethylester, 5,7 Gew.-% Glykolsäuremethylester und 7,0 Gew.-% Methoxyacetyl-glykolsäuremethylester neben Methylacetat und geringen Mengen (etwa 2 Gew.-%) anderer Produkte wie Essigsäure, Dimethylether und Methanol. Der Methylalumsatz beträgt 99% und die Selektivität zu den CO-Insertionsprodukten insgesamt 91%, bezogen auf umgesetztes Methylal.

100 g des rohen Reaktionsgemisches, welches etwa 5 g des bei der Carbonylierung verwendeten Ionenaustauschers enthält, wird nach Zusatz von 100 g Methanol 4 Stunden am Rückfluss erhitzt. Dabei wird der ursprünglich vorhandene Methoxyacetyl-glykolsäuremethylester durch Alkoholyse quantitativ in Methoxyessigsäuremethylester und Glykolsäuremethylester überführt. Fraktionierte Destillation des vom Ionenaustauscher abfiltrierten Reaktionsgemischs gibt 42,5 g Methoxyessigsäuremethylester (Kp 130°C) und 13,0 g Glykolsäuremethylester (Kp 63°C/28 mbar).

Beispiel 3

Ein 200-ml-Autoklav wird mit 50 g Formaldehyd-diethylacetal und 10 g eines perfluorsulfonierten Polymethylens in $H^+$-Form (Austauschkapazität 1.4 mval/g) gefüllt. Man spült mit Stickstoff und gibt 225 bar Kohlenmonoxid zu. Der Autoklav wird 3 Stunden auf 165°C erhitzt, wobei verbrauchtes Kohlenmonoxid ergänzt wird. Nach dem Erkalten wird entspannt und das Reaktionsgemisch gaschromatographisch analysiert. Es enthält 38,1 Gew.-% Glykolsäureethylester. Der Umsatz an Formaldehyd-diethylacetal beträgt 72% und die Selektivität zu Ethoxyessigsäureethylester 45% sowie zu Glykolsäureethylester 23%.

Beispiel 4

Ein 200 ml-Schüttelautoklav wird mit 50 g Formaldehyd-dibutylacetal und 10 g eines Ionenaustauscherharzes in $H^+$-Form (Wassergehalt 3,5 Gew.-%, Austauscherkapazität 4.3 mval/g) gefüllt. Man verfährt weiter wie in Beispiel 1 beschrieben. Der Umsatz an Formaldehyd-dibutylacetal beträgt 68% bei einer Selektivität von 48% zu Butoxyessigsäurebutylester und von 21,3% zu Glykolsäurebutylester.

**Patentansprüche**

1. Verfahren zur Umsetzung von Formaldehyd-acetalen der allgemeinen Formel $RO-CH_2-OR$, worin die beiden Reste R gleich oder verschieden sein können und Alkyl-, Alkoxyalkyl-, Cycloalkyl- oder Phenylalkylgruppen mit bis zu 10 Kohlenstoffatomen bedeuten, mit Kohlenmonoxid in der Flüssigphase bei erhöhten Temperaturen und Drücken, dadurch gekennzeichnet, dass man als Katalysator saure organische Ionenaustauscher mit einer Austauscherkapazität von mehr als 0.5 mval pro Gramm verwendet.

2. Verfahren nach Anspruch 1, dadurch gekennzeichnet, dass man die Umsetzung bei Temperaturen zwischen 50 und 180°C und Drücken zwischen 50 und 300 bar durchführt.

3. Verfahren nach Anspruch 1 oder 2, dadurch gekennzeichnet, dass man die Umsetzung in Gegenwart von organischen Lösungs- oder Verdünnungsmitteln durchführt.

4. Verfahren nach einem der Ansprüche 1 bis 3, dadurch gekennzeichnet, dass man Ionenaustauscher mit einem Wassergehalt von weniger als 5 Gew.-% verwendet.

5. Verfahren nach einem der Ansprüche 1 bis 4, dadurch gekennzeichnet, dass man das bei der Umsetzung mit Kohlenmonoxid entstehende Reaktionsgemisch zusätzlich mit einem Alkohol behandelt.

6. Verfahren nach Anspruch 5, dadurch gekennzeichnet, dass man einen Alkohol der Formel ROH verwendet, wobei der Rest R einem der Reste R im eingesetzten Acetal entspricht.

**Revendications**

1. Procédé pour faire réagir des acétals du formaldéhyde répondant à la formule générale RO–

$CH_2$–OR dans laquelle les deux symboles R représentent chacun, indépendamment l'un de l'autre, un radical alkyle, alcoxy-alkyle, cycloalkyle ou phénylalkyle contenant au plus 10 atomes de carbone, avec le monoxyde de carbone, en phase liquide, à des températures et sous des pressions élevées, procédé caractérisé en ce qu'on utilise, comme catalyseurs, des échangeurs d'ions organiques acides ayant une capacité d'échange supérieure à 0,5 mval par gramme.

2. Procédé selon la revendication 1, caractérisé en ce qu'on effectue la réaction à des températures comprises entre 50 et 180°C et sous des pressions comprises entre 50 et 300 bar.

3. Procédé selon l'une des revendications 1 et 2, caractérisé en ce qu'on effectue la réaction en présence de solvants de diluants organiques.

4. Procédé selon l'une quelconque des revendications 1 à 3, caractérisé en ce qu'on utilise un échangeur d'ions qui a une teneur en eau inférieure à 5% en poids.

5. Procédé selon l'une quelconque des revendications 1 à 4, caractérisé en ce qu'on traite en outre par un alcool le mélange réactionnel qui s'est formé lors de la réaction avec le monoxyde de carbone.

6. Procédé selon la revendication 5 caractérisé en ce qu'on utilise un alcool de formule ROH dans lequel le radical R correspond à l'un des radicaux R de l'acétal mis en jeu.

**Claims**

1. A process for reacting formaldehyde acetals of the general formula RO–$CH_2$–OR in which the two R radicals can be identical or different and denote alkyl, alkoxyalkyl, cycloalkyl or phenylalkyl groups having up to 10 carbon atoms at elevated temperatures and pressures with carbon monoxide in the liquid phase, which process is characterized by using as catalyst acid organic ion exchange materials having an exchange capacity of more than 0.5 mEq per gram.

2. The process as claimed in claim 1, wherein the reaction is carried out at temperatures between 50 and 180°C and under pressures between 50 and 300 bar.

3. The process as claimed in claim 1 or 2, wherein the reaction is carried out in the presence of organic solvents or diluents.

4. The process as claimed in any of claims 1 to 3, wherein the ion exchange material used has a water content of less than 5% by weight.

5. The process as claimed in any of claims 1 to 4, wherein the reaction mixture formed in the reaction with carbon monoxide is additionally treated with an alcohol.

6. The process as claimed in claim 5, wherein the alcohol used has the formula ROH in which the R radical corresponds to one of the R radicals in the acetal used.